# EUROPÄISCHE PATENTSCHRIFT

(11) **EP 0 514 683 B1**
(45) Veröffentlichungstag und Bekanntmachung des Hinweises auf die Patenterteilung: **18.01.1995**
(21) Anmeldenummer: 92107012.4
(22) Anmeldetag: 24.04.1992
(51) Int. Cl.: C07C 17/16

(54) **Verfahren zur Herstellung von Alkyl-, Alkenyl- und Alkinylchloriden**
Process for the preparation of alkyl-, alkenyl and alkinylchlorides
Procédé de préparation de chlorures d'alkyl, d'alkényl et d'alkynyl

(30) Priorität: 18.05.1991 DE 4116365
(43) Veröffentlichungstag der Anmeldung: 25.11.1992
(73) Patentinhaber: BASF Aktiengesellschaft, 67063 Ludwigshafen (DE)
(72) Erfinder: Henkelmann, Jochem, Dr., W-6700 Ludwigshafen (DE); Troetsch-Schaller, Irene, Dr., W-6710 Frankenthal (DE); Wettling, Thomas, Dr., W-6703 Limburgerhof (DE); Kahl, Thomas-Michael, Dr., W-6725 Roemerberg (DE); Hupfer, Leopold, Dr., W-6701 Friedelsheim (DE); Franzischka, Wolfgang, Dr., W-6710 Frankenthal (DE); Koehler, Hermann, Dr., W-6719 Bobenheim (DE)

(56) Entgegenhaltungen:
- EP-A- 0 223 421
- US-A- 4 129 595
- RESEARCH DISCLOSURE, Bd. 219, Juli 1982, Havant, GB; Seiten 243 - 244; "Process for monochlorination of aralkyls"

## Beschreibung

Die vorliegende Erfindung betrifft ein neues Verfahren zur Herstellung von Chloriden durch Umsetzung von Alkyl-, Alkenyl-, Alkinyl-, Alkoxy-, ein- oder zweikernige Aryloxy-, wobei die aromatischen Ringe Heteroatome wie Sauerstoff, Schwefel oder Stickstoff enthalten können und/oder bis zu drei C₁-C₁₂-Alkylgruppen, Halogen oder C₁-C₄-Alkoxygruppen als Substituenten tragen können, Hydroxyalkyl-, Hydroxyalkenyl- und Hydroxyalkinylgruppen tragenden Alkoholen, die gegebenenfalls durch C₁-C₄-Alkylgruppen, C₁-C₄-Estergruppen, Cyan, Halogen, Aryl und Aryloxy substituiert sind, oder von Alkoholen aus der Zuckerreihe mit Phosgen in Gegenwart eines Phosphinoxids als Katalysator.

Die Chloride sind bekannt und eignen sich als Zwischenprodukte für organische Synthesen.

Die US-A 4 129 595 lehrt ein Verfahren zur Herstellung von Chloracetylchlorid durch Umsetzung von Glycolsäure mit Thionylchlorid in Gegenwart einer katalytischen Menge eines Trialkylphosphinoxids.

Aus der GB-A 2 182 039 ist die Chlorierung von Alkoholen mit Phosgen oder Thionylchlorid in Gegenwart von Triarylphosphinoxiden oder Triarylphosphinsulfiden bekannt.

Dieses Verfahren vermag jedoch nicht zu befriedigen, da Triarylphosphinoxide wegen ihrer geringen Reaktivität in überstöchiometrischen Mengen eingesetzt werden müssen und wegen ihrer geringen Löslichkeit die Aufarbeitung des Reaktionsgemisches erschweren.

Der Erfindung lag daher die Aufgabe zugrunde, diesen Nachteilen abzuhelfen.

Demgemäß wurde ein neues Verfahren zur Herstellung von Chloriden durch Umsetzung der entsprechenden Alkohole mit Phosgen in Gegenwart eines Phosphinoxids als Katalysator gefunden, welches dadurch gekennzeichnet ist, daß man aliphatische, cycloaliphatische oder cyclisch-aliphatische Phosphinoxide als Katalysatoren verwendet.

Das erfindungsgemäße Verfahren läßt sich wie folgt veranschaulichen: 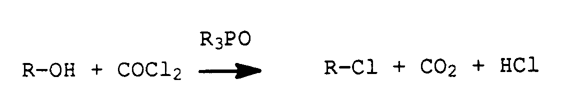
Als im erfindungsgemäßen Verfahren einzusetzende Katalysatoren eignen sich aliphatische, cycloaliphatische und cyclisch-aliphatische Phosphinoxide.

Vorzugsweise verwendet man Trialkylphosphinoxide mit C₁-C₁₂-Alkyl, vor allem C₄-C₈-Alkyl, darunter insbesondere Tributyl-, Trihexyl- und Trioctylphosphinoxid.

Besonders bevorzugt ist ein Gemisch aus Trihexyl-, Dihexyloctyl-, Hexyldioctyl- und Trioctylphosphinoxid.

Bevorzugte cycloaliphatische Phosphinoxide sind Tricycloalkylphosphinoxide mit C₃-C₁₂-Cycloalkyl, vorzugsweise C₅- und C₆-Cycloalkyl wie insbesondere Tricyclohexylphosphinoxid.

Daneben eignen sich aliphatische Bisphosphinoxide und cyclisch-aliphatische Phosphinoxide der allgemeinen Formeln 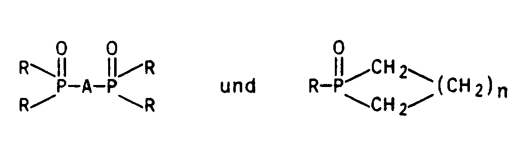
in denen A eine Alkylengruppe mit 2 bis 6, vorzugsweise 4 bis 6 und darunter besonders 4 Brückengliedern bedeutet und die Reste R C₁-C₁₂-Alkylgruppen, vorzugsweise C₁-C₄-Alkylgruppen wie vor allem Methyl und Ethyl oder C₃-C₁₂-Cycloalkylgruppen, vorzugsweise C₅- und C₆-Cycloalkylgruppen wie insbesondere Cyclohexyl bezeichnen. Der Wert n steht vorzugsweise für 2 bis 4, insbesondere für 3.

Man verwendet die aliphatischen, cycloaliphatischen oder cyclisch-aliphatischen Phosphinoxide vorzugsweise in einer Menge von 0,001 bis 0,1 mol, besonders bevorzugt 0,005 bis 0,05 mol pro mol Hydroxylgruppe des Alkohols.

Als erfindungsgemäß einzusetzende Alkohole eignen sich vornehmlich solche der Formel 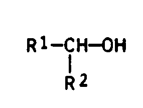
in der
- R¹ und R²: Wasserstoff, Alkyl-, Alkenyl-, Alkinyl-, Alkoxy-, Aryloxy-, Hydroxyalkyl-, Hydroxyalkenyl- oder Hydroxyalkinylgruppen bezeichnen.

Unter den definitionsgemäßen Alkoholen werden im Hinblick auf die gewünschten Verfahrensprodukte solche bevorzugt, in denen die Reste R¹ und R² folgende Bedeutung haben:
- Wasserstoff,
- C₁-C₂₂-Alkyl, vorzugsweise C₄-C₁₈-Alkyl, darunter vorzugsweise C₆-C₁₀-Alkyl wie vor allem n-Hexyl und n-Octyl;
- C₃-C₂₂-Alkenyl, vorzugsweise C₄-C₆-Alkenyl wie insbesondere Butenyl;
- C₃-C₂₂-Alkinyl, vorzugsweise C₃-C₈-Alkinyl wie vor allem Propinyl;
- C₁-C₂₂-Alkoxy, vorzugsweise C₁-C₄-Alkoxy wie insbesondere Propoxy;
- ein- oder zweikernige Aryloxy wie vorzugsweise Phenyloxy, wobei die aromatischen Ringe Heteroatome wie Sauerstoff, Schwefel oder Stickstoff enthalten können und/oder bis zu drei C₁-C₁₂-Alkylgruppen, Halogen wie Fluor, Chlor und Brom oder C₁-C₄-Alkoxygruppen als Substituenten tragen können;
- C₁-C₁₆-Hydroxyalkyl, vorzugsweise C₂-C₁₂-Hydroxyalkyl, darunter vorzugsweise C₄-C₈-Hydroxyalkyl wie vor allem Hydroxybutyl, Hydroxyhexyl und Hydroxyoctyl;
- C₁-C₁₆-Hydroxyalkenyl, vorzugsweise C₂-C₁₂-Hydroxyalkenyl, darunter vorzugsweise C₄-C₈-Hydroxyalkenyl wie insbesondere Hydroxybutenyl, Hydroxyhexenyl und Hydroxyoctenyl;
- C₁-C₁₆-Hydroxyalkinyl, vorzugsweise C₂-C₁₂-Hydroxyalkinyl, darunter vorzugsweise C₄-C₈-Hydroxyalkinyl wie vor allem Hydroxybutinyl, Hydroxyhexinyl und Hydroxyoctinyl.

Hierbei können die Reste außer Wasserstoff ihrerseits Substituenten tragen, vorzugsweise C₁-C₄-Alkyl, C₁-C₄-Ester, Cyan, Halogen wie vor allem Fluor, Chlor und Brom, Aryl wie insbesondere Phenyl und 4-Methoxyphenyl und Aryloxy wie vorzugsweise Phenyloxy.

Bevorzugte Alkohole sind:
- 2-(4-Methoxyphenyl)ethanol-1
- 2-Ethylhexanol-1
- n-Octanol-1
- But-3-en-ol-1
- Propinol
- Butandiol-1,4
- Octandiol-1,8.

Im Hinblick auf die gewünschten Verfahrensprodukte besonders bevorzugt ist Hexandiol-1,6.

Daneben eignen sich Alkohole aus der Zuckerreihe wie 2,3,6,3',4'-Penta-0-acetylsaccharose.

Die Alkohole werden mit Phosgen umgesetzt. Das Molverhältnis von Phosgen zum Alkohol beträgt zweckmäßigerweise 1 bis 1,5 mol, vorzugsweise 1,1 bis 1,2 mol Phosgen pro mol Hydroxylgruppe des Alkohols.

In der Regel führt man die Reaktion bei 40 bis 120°C, vorzugsweise bei 70 bis 90°C durch.

Zweckmäßigerweise wird die Umsetzung bei Normaldruck vorgenommen, jedoch kann man auch bei vermindertem oder erhöhtem Druck arbeiten, also etwa im Bereich von 0,8 bis 5 bar.

Die Reaktionszeiten betragen normalerweise 1 bis 6, meistens 2 bis 3 Stunden.

Es kann zweckmäßig sein, die Umsetzung unter Mitverwendung von Lösungsmitteln durchzuführen. Hierbei eignen sich Alkylester wie Acetessigester und Adipinsäuredimethylester, Arylester wie Benzoesäuremethylester und aromatische Chlorverbindungen wie Dichlorbenzol. Bevorzugt sind aromatische Kohlenwasserstoffe wie Toluol, Xylol und Cumol.

Üblicherweise verwendet man das 1- bis 10-fache, vorzugsweise das 2- bis 5-fache des Alkohols.

Man kann die Reaktion kontinuierlich oder diskontinuierlich vornehmen, bevorzugt ist die diskontinuierliche Fahrweise.

Vorzugsweise verwendet man solche Phosphinoxide, die bei der Reaktionstemperatur flüssig sind und sich in flüssiger Form dosieren lassen. Phosphinoxide, die bei Raumtemperatur flüssig sind, eignen sich daher verfahrenstechnisch besonders gut.

Die Aufarbeitung des Reaktionsgemisches auf die Verfahrensprodukte erfolgt in an sich bekannter Weise, und zwar im allgemeinen durch Destillation, gewünschtenfalls nach Entfernung von überschüssigem Phosgen.

Der katalysatorhaltige Destillationsrückstand kann in die Reaktion rückgeführt werden.

Die nach dem erfindungsgemäßen Verfahren auf wirtschaftliche Weise erhältlichen Chloride sind bekanntermaßen wertvolle Zwischenprodukte für organische Synthesen, insbesondere von Pflanzenschutzmitteln, Galvanohilfsmitteln und Kunststoffvorprodukten.

### Beispiele

### Beispiel 1

### Herstellung von 2-(4-Methoxyphenyl)ethylchlorid

4,4 g (0,02 mol) Tri-n-butyl-phosphinoxid wurden im Verlauf von 15 Minuten bei einer Temperatur von 80°C mit 11 g (0,11 mol) Phosgen versetzt. Nach Erhöhung der Temperatur auf 90 bis 100°C wurden innerhalb 1 Stunde 108 g (1,09 mol) Phosgen und 152 g (1,00 mol) 2-(4-Methoxyphenyl)ethanol zugesetzt. Zur Vervollständigung der Reaktion wurde noch 1 Stunde bei dieser Temperatur gehalten.

Danach wurde das Produkt mittels Durchleiten von Stickstoff bei einer Temperatur von 90°C innerhalb von 2 bis 3 Stunden von überschüssigem Phosgen befreit.

Die anschließende Destillation bei 127 bis 130°C/20 mbar lieferte das 2-(4-Methoxyphenyl)ethylchlorid in einer Ausbeute von 95 bis 99 % und einer Reinheit von 99,6 %.

### Beispiel 2

### Herstellung von 1,6-Dichlorhexan

Analog Beispiel 1 wurden 6,8 g (0,018 mol) Trioctylphosphinoxid mit 11 g (0,11 mol) Phosgen versetzt. Anschließend wurden bei einer Temperatur von 84 bis 91°C weitere 69 g (0,7 mol) Phosgen und 42 g (0,36 mol) Hexandiol-1,6 innerhalb von 2 Stunden zugesetzt sowie eine Stunde bei 88°C die Reaktion vervollständigt.

Nach Entfernung des überschüssigen Phosgens lieferte die anschließende Destillation bei 100°C/30 mbar 1,6-Dichlorhexan in einer Ausbeute von 95 % und einer Reinheit von 99,5 %.

### Beispiel 3

### Herstellung von 1,4-Dichlorbutan

Analog Beispiel 1 wurden 18,6 g (0,085 Tributylphosphinoxid mit 19,7 g (0,2 mol) Phosgen versetzt. Anschließend wurden weitere 350,4 g (3,55 mol) Phosgen und 153,2 (1,7 mol) Butandiol-1,4 zugesetzt. Die anschließende Destillation bei 50°C/100 mbar lieferte das 1,4-Dichlorbutan in einer Ausbeute von 95 % und einer Reinheit von 99,5 %.

## Patentansprüche

1. Verfahren zur Herstellung von Chloriden durch Umsetzung von Alkyl-, Alkenyl-, Alkinyl-, Alkoxy-, ein- oder zweikernige Aryloxy-, wobei die aromatischen Ringe Heteroatome wie Sauerstoff, Schwefel oder Stickstoff enthalten können und/oder bis zu drei C₁-C₁₂-Alkylgruppen, Halogen oder C₁-C₄-Alkoxygruppen als Substituenten tragen können, Hydroxyalkyl-, Hydroxyalkenyl- und Hydroxyalkinylgruppen tragenden Alkoholen, die gegebenenfalls durch C₁-C₄-Alkylgruppen, C₁-C₄-Estergruppen, Cyan, Halogen, Aryl und Aryloxy substituiert sind, oder von Alkoholen aus der Zuckerreihe mit Phosgen in Gegenwart eines Phosphinoxids als Katalysator, dadurch gekennzeichnet, daß man aliphatische, cycloaliphatische oder cyclisch-aliphatische Phosphinoxide als Katalysatoren verwendet.

## Claims

1. A process for preparing chlorides by reaction of alcohols bearing alkyl, alkenyl, alkynyl, alkoxy, mono-or binuclear aryloxy, the aromatic rings being able to contain hetero atoms such as oxygen, sulfur or nitrogen and/or to carry up to three C₁-C₁₂-alkyl groups, halogen or C₁-C₄-alkoxy groups as substituents, hydroxyalkyl, hydroxyalkenyl and hydroxyalkynyl groups, which alcohols are unsubstituted or substituted by C₁-C₄-alkyl groups, C₁-C₄-ester groups, cyano, halogen, aryl and aryloxy, or of alcohols from the sugar series with phosgene in the presence of a phosphine oxide as a catalyst, which comprises using aliphatic, cycloaliphatic or cyclicaliphatic phosphine oxides as catalysts.

## Revendications

1. Procédé de préparation de chlorures par la réaction d'alcools portant des radicaux alkyle, alcényle, alcynyle, alcoxy, aryloxy à un ou deux noyaux, où les cycles aromatiques peuvent contenir des hétéroatomes tels que l'oxygène, le soufre, ou l'azote et/ou porter jusqu'à trois radicaux alkyle en C₁-C₁₂, atomes d'halogène, ou radicaux alcoxy en C₁-C₄ à titre de substituants, hydroxyalkyle, hydroxyalcényle et hydroxyalcynyle, qui peuvent éventuellement être substitués par des radicaux alkyle en C₁-C₄, ester en C₁-C₄, cyano, aryle, aryloxy et des atomes d'halogène, ou bien d'alcools appartenant à la série des sucres, avec le phosgène et en présence d'un oxyde de phosphine servant de catalyseur, caractérisé en ce que l'on utilise des oxydes de phosphines aliphatiques, cycloaliphatiques, ou cycliquement aliphatiques à titre de catalyseurs.
